(19) Europäisches Patentamt
European Patent Office
Office européen des brevets

(11) Veröffentlichungsnummer: **0 210 559 B1**

# (12) EUROPÄISCHE PATENTSCHRIFT

(45) Veröffentlichungstag der Patentschrift: **25.09.91**

(51) Int. Cl.5: **C07D 265/38**, C12Q 1/28

(21) Anmeldenummer: **86109918.2**

(22) Anmeldetag: **19.07.86**

## (54) N-Acyl-dihydroresorufin-Derivate, Verfahren zu deren Herstellung sowie deren Verwendung zur Bestimmung von Wasserstoffperoxid, peroxidatisch wirkenden Verbindungen oder Peroxidase.

(30) Priorität: **25.07.85 DE 3526566**

(43) Veröffentlichungstag der Anmeldung:
**04.02.87 Patentblatt 87/06**

(45) Bekanntmachung des Hinweises auf die Patenterteilung:
**25.09.91 Patentblatt 91/39**

(84) Benannte Vertragsstaaten:
**AT BE CH DE FR GB IT LI LU NL SE**

(56) Entgegenhaltungen:
**EP-A- 0 038 205**
**EP-A- 0 045 220**
**EP-A- 0 124 287**
**EP-A- 0 138 481**

(73) Patentinhaber: **BOEHRINGER MANNHEIM GMBH**
**Sandhofer Strasse 116**
**W-6800 Mannheim 31(DE)**

(72) Erfinder: **Klein, Christian, Dr.**
**Blütenstrasse 16**
**W-8120 Weilheim(DE)**
Erfinder: **Von der Eltz, Herbert, Dr. rer. nat.**
**Kerschensteiner Strasse 18**
**W-8120 Weilheim(DE)**
Erfinder: **Herrmann, Rupert, Dr. rer. nat.**
**In der Au 23**
**W-8120 Weilheim(DE)**
Erfinder: **Junius, Martina, Dr. rer. nat.**
**Eichenstrasse 4**
**W-8139 Bernried(DE)**

EP 0 210 559 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Reaktion von Wasserstoffperoxid oder peroxidatisch wirkenden Substanzen mit Oxidationsindikatoren unter Katalyse von Peroxidase spielt in der analytischen Chemie eine besondere Rolle. Sie gestattet nicht nur den Nachweis von Wasserstoffperoxid oder peroxidatisch wirkenden Verbindungen sowie von Peroxidase oder von Substanzen mit Peroxidase-Aktivität, wie z. B. Hämoglobin, sondern auch die Bestimmung einer Reihe von Stoffen, die mit Sauerstoff in Gegenwart von entsprechenden Oxidasen unter Bildung von Wasserstoffperoxid oder peroxidatisch wirkenden Verbindungen reagieren, und schließlich auch die Bestimmung dieser Oxidasen selbst. Als Beispiele seien einige Verbindungen aufgezählt, deren entsprechende Oxidasen in Klammern stehen:

Glucose (Glucose-Oxidase), Galactose (Galactose-Oxidase), D-Aminosäuren (D-Aminosäure-Oxidase), Cholesterin (Cholesterin-Oxidase), Xanthin (Xanthin-Oxidase), Harnsäure (Uricase), Glycerin (Glycerin- Oxidase), Pyruvat (Pyruvat-Oxidase), Sarkosin (Sarkosin-Oxidase).

Solche Bestimmungen werden üblicherweise derart durchgeführt, daß Wasserstoffperoxid in Gegenwart von Peroxidase mit einem Chromogen stöchiometrisch zu einem Farbstoff reagiert. Die Absorption der Reaktionsmischung wird photometrisch gemessen und ist ein Maß für die Menge des umgesetzten Wasserstoffperoxids und damit der zu bestimmenden Verbindung.

Die Nachweisreaktionen werden vorzugsweise entweder in Küvetten oder mit Hilfe von Trockenreagenzträgern durchgeführt. In letzterem Fall erfolgt die Quantifizierung beispielsweise mit Photometern über eine Transmissionsmessung, mit Remissionsphotometern über Remissionsmessung oder mit Hilfe von Vergleichsfarben durch visuellen Vergleich.

Der Peroxidase-Nachweis ist ferner bei immunologischen Testverfahren z. B. ELISA (enzyme linked immunosorbent assay), erforderlich, bei denen Peroxidase als Markerenzym verwendet wird. Bei solchen immunologischen Testverfahren liegt die Peroxidase-Konzentration üblicherweise in der Größenordnung von $< 10^{-5}$ M.

In der Literatur sind für die vorstehend genannten Methoden zahlreiche Verbindungen bekannt, die als Indikatoren für den Nachweis von Wasserstoffperoxid oder Peroxidase verwendet werden können. Es seien hier als Beispiele genannt: Benzidin und Benzidinderivate, Dichlorphenolindophenol, Aminocarbazole oder Farbstoffe, die als Produkt der oxidativen Kupplung von 4-Aminoantipyrin oder verwandten Stoffen mit Phenolen, Naphtholen, Anilinderivaten oder anderen Kupplungskomponenten entstehen.

Beispielsweise werden in der EP-A-0 038 205 und in der EP-A-0 045 220 chromogene Verbindungen genannt, die zum chemischen Nachweis von Peroxiden geeignet sind; in der EP-A-0 124 287 sind chromogene Verbindungen publiziert, die zum enzymatischen Nachweis von Peroxiden verwendet werden können.

Trotz der Vielzahl bekannter Redoxindikatoren zum Nachweis von Wasserstoffperoxid, peroxidativ wirkender Verbindungen oder Peroxidase wird aber immer noch nach solchen Verbindungen gesucht, die breit, d. h. in den unterschiedlichsten enzymatischen Testsystemen, einsetzbar sind, innerhalb des für enzymatische Tests meist verwendeten pH-Bereichs von etwa neutral bis schwach alkalisch eine pH-unabhängige, hohe Empfindlichkeit besitzen und für die verschiedensten Verfahren, wie z. B. UV-photometrische, visuelle und fluorimetrische Messungen, gut geeignet sind.

Aufgabe der Erfindung war es, geeignete Peroxidasesubstrate bereitzustellen, welche die vorstehenden Anforderungen erfüllen. Gelöst wird diese Aufgabe durch die neuen, erfindungsgemäßen N-Acyl-dihydroresorufin-Derivate.

Gegenstand der Erfindung sind demnach Verbindungen der allgemeinen Formel I

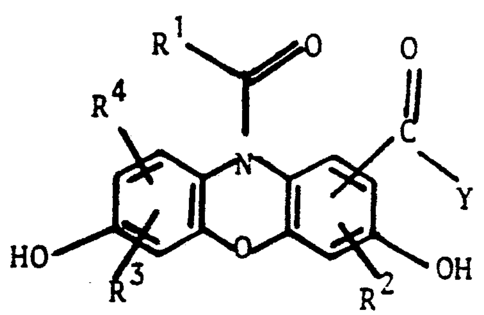

(I)

in der

| | |
|---|---|
| $R^1$ | einen Niederalkyl-, einen Phenyl- oder Naphthylrest oder Aralkylgruppe deren Arylteil eine Phenyl oder Naphthylgruppe und deren Alkylteil 1 bis 5 Kohlenstoffatome enthält, die durch Carboxyl- oder Sulfonsäurereste substituiert sein können, |
| $R^2$, $R^3$ und $R^4$, | die gleich oder verschieden sein können, Wasserstoff, Halogen, eine Niederalkyl- oder eine Niederalkoxygruppe |
| Y | die Gruppe $-NR^5R^6$ oder $-OR^7$, wobei |
| $R^5$ und $R^6$ | jeweils Wasserstoff oder eine Niederalkylgruppe, die durch Carboxyl- oder Sulfonsäurereste substituiert sein kann, oder zusammen eine gegebenenfalls durch Heteroatome unterbrochene Kohlenwasserstoffbrücke und |
| $R^7$ | eine Niederalkylgruppe, die durch eine Niederalkoxy- oder eine Polyniederalkoxygruppe aus 2 bis 5 Niederalkoxyeinheiten substituiert sein kann, |

bedeuten, wobei die Niederalkyl- bzw. Niederalkoxygruppen 1 bis 7 Kohlenstoffatome aufweisen.

Unter einer Niederalkyl- bzw. Niederalkoxygruppe in den Definitionen der Substituenten $R^1$, $R^2$, $R^3$, $R^4$, $R^5$, $R^6$ und $R^7$ sind gesättigte geradkettige oder verzweigte Kohlenwasserstoffreste mit 1 bis 7, vorzugsweise 1 bis 4 Kohlenstoffatome zu verstehen. Besonders bevorzugt sind Methyl- und Ethyl- bzw. Methoxy- und Ethoxygruppen.

Die Polyniederalkoxygruppe in der Definition des Substituenten $R^7$ besteht vorzugsweise aus 2 bis 3 Niederalkoxyeinheiten.

Der Alkylteil der Aralkylgruppe in der Definition von $R^1$ enthält vorzugsweise 1 bis 3 Kohlenstoffatome. Als Aralkylgruppe ist der Benzylrest ganz besonders bevorzugt.

Die Niederalkyl-, Aryl- und auch die Aralkylgruppe in der Definition der Substituenten $R^1$, $R^5$ und $R^6$ können jeweils mehrfach durch Carboxy- und Sulfonsäurereste substituiert sein. Bevorzugt sind Reste, die 1 bis 3 solche Substituenten tragen.

Unter Halogen in der Definiton der Reste $R^2$, $R^3$ und $R^4$ ist Fluor, Chlor, Brom und Jod, vorzugsweise Chlor und Brom zu verstehen.

Die gegebenenfalls durch Heteroatome unterbrochene Kohlenwasserstoffbrücke, die durch die Substituenten $R^5$ und $R^6$ gebildet werden kann, enthält vorzugsweise 2 bis 5, bevorzugt 3 bis 4 Kohlenstoffatome. Die Kohlenwasserstoffbrücke kann durch 1 bis 3 Heteroatome, ausgewählt aus der Gruppe Stickstoff, Sauerstoff und Schwefel unterbrochen sein. Ganz besonders bevorzugt sind der Morpholin- und Piperazin-Rest.

Die Verbindungen der allgemeinen Formel I sind neu. Sie können nach an sich bekannten Methoden hergestellt werden. Ein bevorzugtes Verfahren zur Herstellung der Verbindungen der allgemeinen Formel I ist dadurch gekennzeichnet, daß man Verbindungen der allgemeinen Formel II a und II b

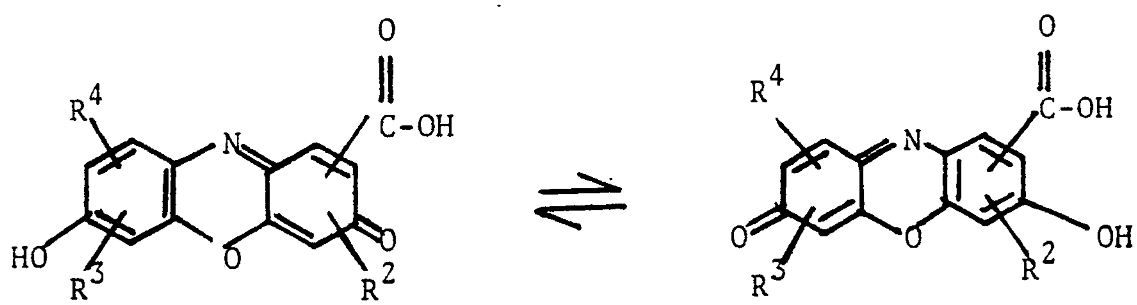

(IIa) (IIb)

in denen

$R^2$, $R^3$ und $R^4$ die in der allgemeinen Formel I angegebene Bedeutung haben,

reduziert und acyliert zu Verbindungen der allgemeinen Formel III

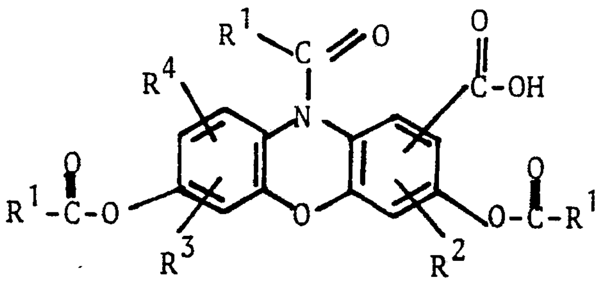

(III)

in der

$R^1$, $R^2$, $R^3$ und $R^4$ die in Formel I angegebene Bedeutung haben,

letztere, gegebenenfalls nach Umwandlung der Carbonsäurefunktion in ein reaktives Carbonsäurederivat, mit Verbindungen der allgemeinen Formel IV

HY (IV)

in der

Y die in Formel I angegebene Bedeutung hat,

umsetzt und anschließend die O-Acylgruppen selektiv abspaltet.

Die Verbindungen der allgemeinen Formel II a oder II b sind entweder bekannte Substanzen, die sich von Resorufin ableiten, oder sie lassen sich in Analogie zu bekannten Verbindungen herstellen.

Hierzu werden in vorteilhafter Weise Nitrosoresorcin-Derivate der allgemeinen Formel V

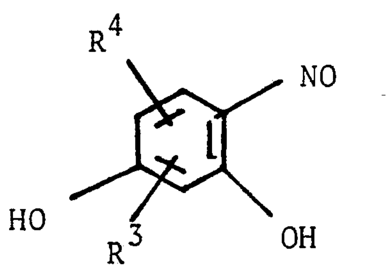

(V)

in der

$R^3$ und $R^4$ die in der allgemeinen Formel I angegebene Bedeutung besitzen,

mit Resorcin-Derivaten der allgemeinen Formel VI

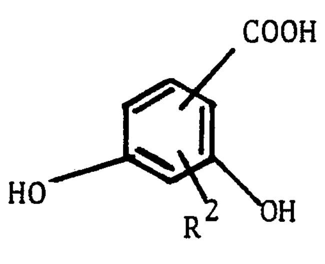

(VI)

in der

$R^2$ die in der allgemeinen Formel I angegebene Bedeutung hat,

in Anwesenheit von Braunstein und Schwefelsäure bei niedrigen Temperaturen umgesetzt werden. Es entstehen dabei zunächst Resazurin-Derivate, bei denen das N-Atom noch ein Sauerstoffatom trägt. Diese Verbindungen lassen sich leicht mit Zinkpulver in Anwesenheit von Ammoniak in Verbindungen der allgemeinen Formel II a oder II b überführen.

Die Reaktion der Verbindungen der allgemeinen Formel V mit Verbindungen der allgemeinen Formel VI wird üblicherweise bei einer Temperatur zwischen -10 bis 50° C, vorzugsweise zwischen 0 und 30° C

durchgeführt. Besonders schonend verläuft die Umsetzung, wenn man die Substanzen der allgemeinen Formel V und der Formel VI bei ungefähr 0° C vermischt und das Reaktionsgemisch sich anschließend auf Raumtemperatur erwärmen läßt. Die Konzentration an Braunstein sollte zweckmäßigerweise bei 0,5 bis 5, vorzugsweise 1 bis 2 mol/l liegen. Die Schwefelsäurekonzentration sollte 0,5 bis 5, vorzugsweise 1 bis 3 mol/l betragen.

Die Reduktion der zunächst entstehenden Resazurin-Derivate zu Verbindungen der allgemeinen Formel II a oder II b wird vorzugsweise in ammoniakalischer Lösung mit Zinkstaub durchgeführt (vgl. Nietzki et al., Ber. Dtsch. Chem. Ges. 22, 3020 [1889]) oder mit Natriumborhydrid. Als Lösungsmittel verwendet man zweckmäßigerweise Wasser-Alkohol-Gemische, bevorzugt ein Gemisch aus 1 Teil Wasser mit 0 bis 4 Teilen Methanol. Pro Mol zu reduzierender Substanz werden 1 bis 20, vorzugsweise 1 bis 5 Mol Zinkstaub bzw. Natriumborhydrid portionsweise zugegeben. Die Temperatur der Reaktionslösung wird dabei bei -10 bis +35° C, vorzugsweise bei +5 bis +10° C gehalten. Die genaue Einhaltung des Temperaturbereiches hat sich als notwendig für einen eindeutigen Reaktionsverlauf erwiesen. Ohne Kühlung führt die exotherme Reaktion zu Nebenprodukten, die schwer abtrennbar sind.

Unter den gewählten milden Bedingungen verläuft die Umsetzung zwischen den Substanzen der allgemeinen Formel V und der allgemeinen Formel VI eindeutig und mit guter Ausbeute. Der gewählte Syntheseweg ist variationsfähig. Dies eröffnet insbesondere im Hinblick auf die Darstellung unsymmetrisch substituierter Resorufin-Derivate zahlreiche Synthesemöglichkeiten.

Zur Herstellung der Triacyl-Derivate der allgemeinen Formel III werden die entsprechenden Resorufin-Derivate der allgemeinen Formel II a und II b zunächst mit Zinn-II-chlorid, Chrom-II-acetat, oder einem äquivalenten Reduktionsmittel, oder elektrochemisch reduziert. Zur Reduktion erwärmt man das Resorufin-Derivat 10 min bis 1 Stunde mit 2 bis 10, bevorzugt 2 bis 6 Äquivalenten des Reduktionsmittels in einem geeigneten Lösungsmittel, vorzugsweise Zinn-II-chlorid in 5 bis 35 %iger wäßriger Salzsäure. Beim Abkühlen fällt die Dihydroverbindung aus. Die Acylierung erfolgt in üblicher Weise mit einem geeigneten Acylierungsmittel, wie Säureanhydrid oder Säurechlorid, z.B. Acetanhydrid, Benzoylchlorid . Bevorzugt werden die Verbindungen der allgemeinen Formel III in einem Eintopfverfahren durch reduktive Acylierung der Resorufin-Derivate II a und II b hergestellt. Das entsprechende Resorufin-Derivat wird hierzu mit 2 bis 6 Äquivalenten des Reduktionsmittels in Gegenwart des Acylierungsmittels in einem geeigneten Lösungsmittel 5 min bis 3 Stunden unter Rückfluß erhitzt oder bei Raumtemperatur 4 bis 16 Stunden gerührt.

Zur Umsetzung mit den Verbindungen IV ist es zweckmäßig, die Carbonsäuren der Formel III in reaktive Carbonsäurederivate, wie Carbonsäurechloride, -ester oder -anhydride, zu überführen. Hierzu stehen dem Fachmann zahlreiche literaturbekannte Verfahren zur Verfügung. Besonders bevorzugt ist die Umwandlung der Carbonsäurefunktion in ein Carbonsäurechlorid, z.B. mit Oxalylchlorid/Dimethylformamid oder Thionylchlorid/Dimethylformamid.

Die Verbindungen der allgemeinen Formel IV sind Amine bzw. Alkohole, mit denen die Carbonsäurederivate der allgemeinen Formel III bzw. deren reaktive Derivate in Carbonsäureamide bzw. -ester, z.B. in Gegenwart eines Acylierungskatalysators, wie Dimethylaminopyridin, überführt werden.

Besonders bevorzugte Amine der allgemeinen Formel HY sind solche mit einer polaren Gruppe, wie beispielsweise Morpholin, Methoxyethylamin oder Glycinamid, weil dadurch die Wasserlöslichkeit der entsprechenden Leucoresorufin-Derivate erhöht wird. Ebenso können Aminocarbonsäuren eingesetzt werden. Es ist zweckmäßig, funktionelle Gruppen, die nicht an der gewünschten Umsetzung beteiligt sind, in üblicher Weise zu schützen. Beispiele für solche geschützten Aminocarbonsäuren sind Glycin-tert.-butylester, Glycinbenzylester oder N -BOC-Lysinmethylester. Die eingeführten Schutzgruppen werden nach erfolgter Umsetzung wieder in bekannter Weise abgespalten.

Als Alkohole der allgemeinen Formel HY eignen sich im Prinzip alle möglichen Alkohole. Besonders bevorzugt sind Diethylenglycol-monoethylether und Triethylenglycol-monoethylether sowie die entsprechenden Monomethylether.

Zur Herstellung der erfindungsgemäßen N-Acylderivate der allgemeinen Formel I müssen nach der Umsetzung der Carbonsäuren III mit Aminen bzw. Alkoholen IV die beiden O-Acylgruppen selektiv abgespalten werden. Dies wird durch Reaktion mit 2 - 10 mol, bevorzugt 2 - 4 mol Natriumsulfit in einem Gemisch aus Wasser und einem wasserlöslichen Lösungsmittel wie 1,4-Dioxan, Methanol oder Ethanol, bevorzugt Wasser/1,4-Dioxan 1:1 erreicht. Die Reaktionstemperatur beträgt 20 - 100° C, bevorzugt 80 - 100° C. Unter diesen Reaktionsbedingungen lassen sich die beanspruchten N-Acyl-dihydroresorufine der allgemeinen Formel I in hohen Ausbeuten herstellen.

Die N-Acyl-dihydroresorufine der allgemeinen Formel I lassen sich leicht durch Wasserstoffperoxid in Gegenwart von Peroxidase zu farbigen und fluoreszierenden Resorufin-Derivaten oxidieren.

Ein weiterer Gegenstand der Erfindung ist somit die Verwendung der Substanzen der allgemeinen Formel I zum Nachweis von Wasserstoffperoxid (oder von peroxidatisch wirkenden Verbindungen), von

Peroxidase (oder von Substanzen mit Peroxidase-Aktivität).Über solche Nachweisreaktionen ist es selbsverständlich auch möglich, solche Stoffe, die mit Sauerstoff in Gegenwart von entsprechenden Oxidasen unter Bildung von Wasserstoffperoxid bzw. von peroxidatisch wirkenden Verbindungen reagieren, sowie Enzymaktivitäten in Wasserstoffperoxid bzw. peroxidatisch wirkende Verbindungen produzierenden Systemen zu bestimmen.

Die entstehenden Oxidationsprodukte der N-Acyl-dihydroresorufin-Derivate sind außer für UV-photometrische und visuelle Detektion vor allem auch für fluorimetrische Messungen geeignet. Dies ist deshalb von Bedeutung, weil gegenüber photometrischen Methoden die Empfindlichkeit fluorometrischer Bestimmungen oft um einige Zehnerpotenzen erhöht ist. In manchen Fällen muß mit fluorogenen Substraten gearbeitet werden, beispielsweise bei der Untersuchung enzymatischer Aktivitäten in Zellen mit automatischen Geräten zur Zelldifferenzierung (Zytofluorometrie) sowie bei der Analyse immobilisierter Enzyme mit Durchflußmikrofluorometrie. In anderen Fällen, z. B. bei der Bestimmung der enzymatischen Markierung von Testsystemen (Enzymimmunoassays), die oft mit Peroxidase durchgeführt wird, wird der Multiplikationseffekt der enzymatischen Katalyse durch Verwendung fluorogener Substrate beträchtlich verstärkt.

Sowohl die Lichtabsorption im UV- und sichtbaren Bereich als auch die Fluoreszenzintensität der Oxidationsprodukte der erfindungsgemäßen Verbindungen der allgemeinen Formel I sind im neutralen bis schwach alkalischen pH-Bereich praktisch pH-unabhängig. Da der pH-Wert der Lösung je nach dem zu untersuchenden Enzymsystem optimiert werden muß, um maximale Aktivität zu erreichen, und damit innerhalb eines Bereichs von 6,5 bis 9,5 variieren kann, ist die pH-Unabhängigkeit der Lichtabsorption und der Fluoreszensintensität zur Erzielung einer vergleichbar hohen Empfindlichkeit auch in unterschiedlichen enzymatischen Tests sehr wichtig.

Besonders hervorzuheben ist in diesem Zusammenhang die gute Wasserlöslichkeit der beanspruchten N-Acyl-dihydroresorufin-Derivate in dem erwähnten pH-Bereich. Damit entfällt die Notwendigkeit des Zusatzes organischer Lösungsmittel oder von Detergentien zu enzymatischen Tests, um das verwendete Chromogen in Lösung zu bringen. Dieser Vorteil wirkt sich unter anderem bei kinetischen Enzymtests aus, wo gute Löslichkeiten von Substrat und Produkt erforderlich sind und wo Lösungsmittel- oder Detergenszusätze oft die Enzymaktivitäten beeinflussen.

Zur Bestimmung von Wasserstoffperoxid oder von peroxidatisch wirkenden Verbindungen werden eine Verbindung der allgemeinen Formel I, Peroxidase, ein geeignetes Puffersystem sowie gegebenenfalls weitere Reagenzien und Hilfsstoffe mit der Probe, die den zu bestimmenden Stoff enthält, vermischt. Die N-Acyl-dihydroresorufin-Derivate der allgemeinen Formel I werden dabei oxidiert. Die dadurch bewirkte Änderung der Absorption bzw. Fluoreszensintensität der Reaktionsmischung wird photometrisch bzw. fluorimetrisch gemessen. Durch direkten Vergleich mit einer Standardlösung oder durch indirekten Vergleich mit einer Standardkurve kann so der Gehalt an zu bestimmender Substanz in der Probe ermittelt werden. Es sind sowohl kinetische als auch Endpunkts-Messungen möglich.

Zur Bestimmung von Peroxidase bzw. von Verbindungen mit Peroxidase-Aktivität werden eine Verbindung der allgemeinen Formel I, Wasserstoffperoxid bzw. eine peroxidatisch wirkende Verbindung, ein geeignetes Puffersystem sowie gegebenenfalls weitere Reagenzien und Hilfsstoffe mit der Probe, die den zu bestimmenden Stoff enthält, vermischt. Das erhaltene Reaktionsgemisch wird in vorstehend beschriebener Weise weiterbehandelt.

Die Verbindungen der allgemeinen Formel I können ebenfalls zur Bestimmung von Substanzen, die mit Sauerstoff in Gegenwart entsprechender Oxidasen Wasserstoffperoxid oder peroxidatisch wirkende Verbindungen liefern, oder von Enzymaktivitäten in Wasserstoffperoxid oder peroxidatisch wirkende Verbindungen produzierenden Systemen verwendet werden. Die Bestimmung dieser Substanzen bzw. dieser Enzyme erfolgt in der oben angegebenen Weise, nur daß der Reaktionsmischung zusätzlich die entsprechende Oxidase sowie ggf. weitere Hilfsenzyme bzw. das geeignete Substrat zugegeben werden. Als Beispiele für solche enzymatische Systeme seien die Reaktionen von Glucose zu Gluconolacton mit Glucose-Oxidase, von Cholesterin zu Cholestenon mit Cholesterin-Oxidase, von Harnsäure zu Allantoin mit Uricase oder von Glycerin zu Glyceraldehyd mit Glycerin-Oxidase aufgezählt. Die Konzentrationen solcher Substrate bzw. Enzyme in Körperflüssigkeiten sind wichtige diagnostische Parameter, welche auf die genannte Weise einfach und genau bestimmt werden können.

Ein weiterer Gegenstand der Erfindung ist somit ein Verfahren zur Bestimmung von Wasserstoffperoxid, peroxidatisch wirkenden Verbindungen und von Substanzen, die mit Sauerstoff in Gegenwart entsprechender Oxidasen Wasserstoffperoxid oder peroxidatisch wirkende Verbindungen liefern, sowie ein Verfahren zur Bestimmung von Peroxidase, von Verbindungen mit Peroxidase-Aktivität und von Enzymaktivitäten in Wasserstoffperoxid bzw. peroxidatisch wirkende Verbindungen produzierenden Systemen mit Hilfe von Verbindungen der allgemeinen Formel I. Ferner werden Reagenzien beansprucht, die zur Durchführung der oben genannten Verfahren geeignet sind.

Das Reagenz zum Nachweis von Wasserstoffperoxid, von peroxidatisch wirkenden Verbindungen bzw. von Substanzen, die solche Verbindungen liefern, enthält neben einem oder mehreren der erfindungsgemäßen N-Acyl-dihydroresorufin-Derivate der allgemeinen Formel I Peroxidase sowie weitere für den jeweiligen Parameternachweis notwendigen Enzyme, ein geeignetes Puffersystem sowie gegebenenfalls weitere Reagenzien und Hilfsstoffe, wie beispielsweise Netzmittel, Stabilisatoren, galenische Zusatzstoffe, Gerüstbildner.

Das Reagenz zum Nachweis von Peroxidase, von Verbindungen mit Peroxidase-Aktivität bzw. von Enzymaktivität in Wasserstoffperoxid bzw. peroxidatisch wirkende Verbindungen produzierenden Systemen, enthält neben einem oder mehreren der erfindungsgemäßen N-Acyl-dihydroresorufin-Derivaten der allgemeinen Formel I Wasserstoffperoxid bzw. eine peroxidatisch wirkende Verbindung, gegebenenfalls erforderliche Substrate und Hilfsenzyme, ein geeignetes Puffersystem sowie gegebenenfalls weitere Reagenzien und Hilfsstoffe.

Das erfindungsgemäße Reagenz kann in Form einer Lösung, als Lyophilisat, als Pulvergemisch, Reagenztablette oder, auf ein geeignetes Trägermaterial aufgebracht, vorliegen.

Das erfindungsgemäße Reagenz in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Ethanol, Aceton oder Dimethylformamid in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des Reagenzes in Form eines Lyophilisats wird eine Lösung getrocknet, die neben sämtlichen für den Test benötigten Reagentien übliche Gerüstbildner, wie z. B. Polyvinylpyrrolidon und eventuell weitere Füllstoffe, wie z. B. Mannit, Sorbit oder Xylit enthält.

Ein Reagenz in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 30 bis 200 mg, vorzugsweise 50 bis 80 mg, auf.

Als Trägermaterial können die für Teststreifen bekannten Trägermaterialien, wie Papier-, Glas- oder Kunststoffvliese, Netze und Gewebe aus Fasermaterial oder saugfähige, poröse Filme oder Gele Verwendung finden.

Zur Herstellung des Reagenzes in Form eines Teststreifens wird ein geeignetes Trägermaterial, vorzugsweise Filterpapier, Cellulose oder Kunstfaservlies mit Lösungen der erforderlichen, üblicherweise zur Herstellung von Teststreifen verwendeten Reagentien in leicht flüchtigen Lösungsmitteln, wie z. B. Wasser, Methanol, Ethanol oder Aceton imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des Reagenzes enthalten. So kann beispielsweise in einem ersten Schritt mit einer wäßrigen Lösung, die den Puffer und andere wasserlösliche Zusatzstoffe enthält, und dann in einem zweiten Schritt mit einer Lösung, die das N-Acyl-dihydroresorufin enthält, imprägniert werden. Die fertigen Testpapiere können als solche verwendet werden oder in an sich bekannter Weise an Griffe angeklebt oder vorzugsweise zwischen Kunststoffe und feinmaschige Netzwerke gemäß DBP 2118455 eingesiegelt werden.

Zur Herstellung von Trockenreagenzien aus löslichen Filmbildnern werden aus den Polymeren Lösungen hergestellt, die so viskos sind, daß sich aus ihnen nach den bekannten Herstellungsverfahren wie Rakeln, Vorhandverfahren, Rollcoating usw. Filme herstellen lassen. In diesen Lösungen werden die Reagenzien, Puffersubstanzen, Hilfsstoffe und Reagenzstabilisatoren eingearbeitet. Die Beschichtungsmassen werden auf Trägerfolien aufgebracht, getrocknet und die fertigen Filme zu Teststreifen verarbeitet.

N-Acyl-dihydroresorufin-Derivate der allgemeinen Formel I können auch für immunologische Bestimmungsmethoden eingesetzt werden, bei denen Peroxidase als Indikatorenzym verwendet wird und dessen Aktivität nach Durchführung der immunologischen Reaktion ermittelt werden muß. Solche immunologischen Bestimmungsmethoden mit enzymatischer Indikatorreaktion sind dem Fachmann als Enzymimmunoassays bekannt. Diese Methoden dienen zur Bestimmung der Konzentration von Proteinen, Polysacchariden, Hormonen, Arzneistoffen und anderen niedermolekularen Substanzen im Bereich von $10^{-5}$ bis $10^{-12}$ mol/l. Je nach Erfordernis von Phasentrennschritten unterscheidet man zwischen homogener und heterogener Testführung. Eine weitere Unterteilung kann in kompetitive und nicht-kompetitive Testprinzipien erfolgen. Alle Testprinzipien arbeiten jedoch mit Enzym-Antigen- bzw. Enzym-Antikörper-Konjugaten. Die enzymatische Indikatorreaktion ist allen Enzymimmunoassays gemeinsam. Ein für solche Zwecke geignetes Indikatorenzym ist z. B. Peroxidase. Die Bestimmung der Peroxidase in solchen Enzymimmunoassays erfolgt üblicherweise, indem ein geeignetes Substrat zugesetzt wird, das mit Wasserstoffperoxid enzymatisch

oxidiert und in üblicher Weise photometrisch oder auch fluorimetrisch vermessen wird.

Die nachfolgenden Beispiele zeigen einige Verfahrensvarianten, die zur Synthese der erfindungsgemäßen Verbindungen beschritten werden können sowie beispielhaft die Verwendung der neuen N-Acyl-dihydroresorufin-Derivate der allgemeinen Formel I. Sie sollen jedoch nicht eine Einschränkung des Erfindungsgegenstandes bedeuten.

Beispiel 1

N-Acetyl-dihydroresorufin-4-carbonsäuremorpholid

A) Resorufin-4-carbonsäure

16 g Nitrosoresorcin, 15,5 g 2,6-Dihydroxybenzoesäure und 8,6 g Braunstein werden in 200 ml Methanol suspendiert und auf 0° C gekühlt. Dazu tropft man 10,6 ml konz. Schwefelsäure und rührt dann noch 2 Stunden bei Raumtemperatur. Die ausgefallene rote Resazurin-4-carbonsäure wird abfiltriert, mit Methanol gewaschen und getrocknet.

Das Resazurinderivat wird in 200 ml Wasser und 50 ml 25 %igem Ammoniak aufgenommen und filtriert. Zu dem blauen Filtrat gibt man unter Eiskühlung portionsweise 50 g Zinkstaub und läßt auf Raumtemperatur erwärmen. Der Reduktionsverlauf läßt sich leicht dünnschichtchromatographisch verfolgen (Fließmittel: Methanol/Essigester 1:1; Kieselgel-DC-Platten). Die Reaktionslösung wird filtriert, danach säuert man das Filtrat mit Eisessig und wenig konz. Salzsäure an. Die ausgefallene Resorufin-4-carbonsäure wird abfiltriert und über Sicapent im Vakuum getrocknet.
Ausbeute: 16,33 g
Rf (DC: Kieselgel; Fließmittel: n-Butanol/Eisessig/Wasser 4:1:1): 0.4.

B) N,O,O-Triacetyl-dihydroresorufin-4-carbonsäure

12,9 g Resorufin-4-carbonsäure werden in 30 ml Eisessig und 30 ml Acetanhydrid aufgenommen, mit 27,6 g Zinn-II-chlorid versetzt und 1 Stunde bei 80° C gerührt. Man gibt auf 600 ml Eiswasser, rührt 1 Stunde und filtriert den Niederschlag ab. Nach Trocknung wird der Feststoff in 500 ml Aceton aufgenommen. Man saugt ab, dampft das Filtrat ein und erhält nach Trocknen 11,3 g Produkt.
Rf (DC: Kieselgel; Fließmittel: Chloroform/Methanol/Eisessig 9:1:0,1): 0,46
$^1$H-NMR ([$D_6$]-DMSO): $\delta$ = 2.24, 2.26 und 2.29 (je s, je 3 H);
6.94 (dd, J = 8.5 und 2.2 Hz, 1 H); 6.98 (d, J = 2.2 Hz, 1 H);
7.04 (d, J = 9 Hz, 1 H); 7.61 (d, J = 8.5 Hz, 1 H);
7.67 ppm (d, J = 9 Hz, 1 H).

C) N,O,O-Triacetyl-dihydroresorufin-4-carbonsäuremorpholid

3,85 g N,O,O-Triacetyl-dihydroresorufin-4-carbonsäure werden mit 5,4 ml Oxalylchlorid versetzt und auf 0° C gekühlt. Dazu gibt man einen Tropfen Dimethylformamid und läßt das Reaktionsgemisch unter Rühren auf Raumtemperatur erwärmen. Das Edukt löst sich dabei unter Gasentwicklung. Man dampft bei Raumtemperatur im Vakuum zur Trockne ein, nimmt je dreimal in 20 ml trockenem Methylenchlorid auf und dampft wiederum zur Trockne ein. Man erhält so 4 g N,O,O-Triacetyl-dihydroresorufin-4-carbonsäureohlorid, das als Rohprodukt weiter verarbeitet wird.

Das Säurechlorid wird in 50 ml trockenem Methylenchlorid gelöst. Dazu tropft man 3,1 ml Triethylamin und anschließend 1,2 ml Morpholin. Nach 2 Stunden Rühren wäscht man die Reaktionslösung mit Wasser, gesättigter Natriumhydrogencarbonatlösung und verdünnter Salzsäure, trocknet die Methylenchlorid-Phase über Magnesiumsulfat und dampft ein. Der Rückstand wird aus Ethanol kristallisiert.
Ausbeute: 8,1 g
$^1$H-NMR ([$D_6$]-DMSO): $\delta$ = 2.20 - 2.21 (3 s, 9 H); 3.50 - 3.80 (m, 8 H);
6.70 - 7.05 (m, 2 H); 7.12 (d, J = 2 Hz, 1 H); 7.54 (d, J = 9 Hz, 1 H);
7.60 ppm (d, J = 9 Hz, 1 H).

D) N-Acetyl-dihydroresorufin-4-carbonsäuremorpholid

2,02 g N, O, O-Triacetyl-dihydroresorufin-4-carbonsäuremorpholid werden mit 1,2 g Natriumsulfit (wasserfrei) 0,5 Stunden in 50 ml Dioxan/Wasser 1:1 unter Rückfluß erhitzt. Man dampft ein, nimmt mit 50

ml Aceton auf, filtriert und dampft das Filtrat wiederum ein. Man chromatographiert an Kieselgel (Eluens Chloroform/Methanol 8:2) und erhält 1,0 g Produkt.
Rf (DC: Kieselgel; Fließmittel: Chloroform/Methanol 8:2): 0,8.

$^1$H-NMR ([$D_6$]-DMSO): δ = 2.20 (s, 3 H); 3.10 - 3.16 (m, 2 H); 3.4 - 3.8 (m, 6 H); 6.48 (d, J = 2.4 Hz, 1 H); 6.53 (dd, J = 9.8 und 2.4 Hz, 1 H); 6.62 (d, J = 9 Hz, 1 H); 7.30 (d, J = 9.8 Hz, 1H); 7.33 (d, J = 9.8 Hz, 1H); 9.62 (s, 1 H); 9.91 ppm (s, 1 H).

UV/VIS (0,1M Kaliumphosphatpuffer, pH 7,5) : $\lambda_{max}$ = 200 nm
nach Oxidation mit $H_2O_2$/Peroxidase : $\lambda_{max}$ = 575 nm
Fluoreszenzemission : $\lambda_{max}$ = 590 nm

In analoger Weise erhält man:

a) N-Acetyl-6-methyldihydroresorufin-4-carbonsäuremorpholid
aus 2-Methyl-4-nitrosoresorcin über
6-Methyl-resazurin-4-carbonsäure und
6-Methyl-resorufin-4-carbonsäure.

$^1$H-NMR ([$D_6$]-DMSO): δ 2.17 und 2.23 (je s, 6H); 3.5-3.8 (m, 8H); 6.20 (d, J = 8.6 Hz, 1H); 6.6 (d, J = 8.8 Hz, 1H); 6.79 (d, J = 8.8 Hz,1 1H); 7.35 ppm (d, J = 8.8 Hz, 1H)

UV/VIS (0.1M Kaliumphosphatpuffer, pH 8.0):
nach Oxidation mit $H_2O_2$/Peroxidase : $\lambda_{max}$= 585 nm

b) N-Acetyl-8-ethyldihydroresorufin-4-carbonsäuremorpholid
aus 4-Ethyl-6-nitrosoresorcin über
8-Ethyl-resazurin-4-carbonsäure und
8-Ethyl-resorufin-4-carbonsäure.

DC (Kieselgel, Fließmittel: Essigester) : Rf = 0,38
UV/VIS (0,1M Kaliumphosphatpuffer pH 8.0) :
nach Oxidation mit $H_2O_2$/Peroxidase : $\lambda_{max}$= 575 nm,
Fluoreszenzemission : $\lambda_{max}$= 598 nm

c) N-Acetyl-8-chlorresorufin-1-carbonsäuremorpholid
aus 4-Chlor-6-nitrosoresorcin über
8-Chlor-resazurin-1-carbonsäure und
8-Chlor-resorufin-1-carbonsäure

UV/VIS (0.1M Kaliumphosphatpuffer, pH 8.0):
nach Oxidation mit $H_2O_2$/Peroxidase : $\lambda_{max}$ = 570 nm

d) N-Acetyl-6,8-dichlordihydroresorufin-4-carbonsäure-morpholid
aus 2,4-Dichlor-6-nitrosoresorcin über
6,8-Dichlor-resazurin-4-carbonsäure und
6,8-Dichlor-resorufin-4-carbonsäure

e) N-Acetyl-8-bromdihydroresorufin-4-carbonsäure-morpholid
aus 4-Brom-6-nitrosoresorcin über

8-Brom-resazurin-4-carbonsäure
8-Brom-resorufin-4-carbonsäure

Beispiel 2

N-Acetyl-dihydroresorufin-4-carbonsäure-(carboxymethyl)amid

A) N,O,O-Triacetyl-dihydroresorufin-4-carbonsäure-t-butoxycarbonylmethylamid

10.7 g N,O,O-Triacetyl-dihydroresorufin-4-carbonsäure (hergestellt nach Beispiel 1 b) werden, wie in Beispiel 1 c) beschrieben, mit 27 ml Oxalylchlorid in das Säurechlorid überführt.

Das Säurechlorid wird in 100 ml Methylenchlorid aufgenommen. Dazu tropft man 7.8 g in 20 ml Methylenchlorid gelösten Glycin-t-butylester und rührt 3 Stunden bei Raumtemperatur. Man wäscht mit gesättigtem Natriumhydrogencarbonat, 1 N Citronensäure und Wasser. Nach Trocknen über Magnesiumsulfat dampft man die organische Phase ein, nimmt in wenig Essigester auf und filtriert über Kieselgel mit Essigester als Eluens. Man erhält 11 g leicht bräunlich gefärbtes Produkt.

Rf (DC: Kieselgel, Fließmittel: Essigester): 0,68.

B) N-Acetyl-dihydroresorufin-4-carbonsäure-t-butoxycarbonylmethylami

9 g N,O,O-Triacetyl-dihydroresorufin-4-carbonsäure-t-butoxycarbonylmethylamid werden mit 4,6 g wasserfreiem Natriumsulfit 30 min in 100 ml Dioxan/Wasser 1:1 unter Rückfluß erhitzt. Man dampft ein, nimmt den Rückstand in 250 ml Aceton auf, filtriert und dampft das Filtrat wiederum ein. Durch Kristallisation aus Ethanol erhält man 4,5 g Produkt. 1,4 g davon werden an Kieselgel mit Methylenchlorid/Methanol 20:1 chromatographiert.

Ausbeute: 0,9 g

Rf (DC: Kieselgel, Fließmittel: Essigester): 0,77

$^1$H-NMR ([$D_6$]-DMSO): $\delta$ = 1.49 (s, 9 H); 2.20 (s, 3 H); 4.07 (d, J = 6 Hz, 2 H);
6.61 (dd, J = 9.8 und 2.4 Hz, 1 H); 6.69 (d, J = 9.8 Hz, 1 H);
6.86 (d, J = 2.4 Hz, 1 H); 7.35 (d, J = 9.8 Hz, 1 H) 7.51 (d, J = 9.8 Hz, 1 H);
8.77 (t, breit, J = 6 Hz, 1 H);
9.74 (s, 1 H); 12.61 (s, 1 H).

C) N-Acetyl-dihydroresorufin-4-carbonsäure-(carboxymethyl)amid

500 mg N-Acetyldihydroresorufin-4-carbonsäure-t-butoxycarbonylmethylamid werden 30 min in 10 ml Trifluoressigsäure stehengelassen. Man tropft langsam 40 ml Wasser zu, filtriert, wäscht mit Wasser und trocknet.

Ausbeute: 410 mg.

Rf (DC: Kieselgel, Fließmittel: n-Butanol/Eisessig/Wasser 4:1:1): 0,79

$^1$H-NMR: ([$D_6$]-DMSO): $\delta$ = 2.20 (s, 3 H); 4.08 (d, J = 6 Hz, 1 H);
6.61 (dd, J = 9.8 und 2.4 Hz, 1 H); 6.69 (d, J = 9.8 Hz, 1 H);
6.89 (d, J = 2.4 Hz, 1 H); 7.34 und 7.52 (je d, J = 9.8 Hz, 2 H);
8.81 (t, breit, J = 6 Hz, 1 H); 9.74 (s, breit, 1 H);
12,5 ppm (s, 1 H).

UV/VIS (0.1M Kaliumphosphatpuffer, pH 7,5) : $\lambda_{max}$ = 204 nm
nach Oxidation mit $H_2O_2$/Peroxidase : $\lambda_{max}$ = 571 nm
Fluoreszenzemission : $\lambda_{max}$ = 588 nm

In analoger Weise erhält man:

a) aus N,O,O,-Triacetyldihydroresorufin-4-carbonsäure und Sarcosin-t-butylester über
N,O,O-Triacetyl-dihydroresorufin-4-carbonsäure-(t-butoxycarbonylmethyl)-methylamid
Rf (DC: Kieselgel, Fließmittel: Chloroform/Methanol/Eisessig 9:1:0.1): 0.77,
N-Acetyl-dihydroresorufin-4-carbonsäure-(t-butoxycarbonylmethyl)-methylamid
Rf (DC s.o.) : 0.56,
N-Acetyl-dihydroresorufin-4-carbonsäure-(carboxymethyl)-methylamid
Rf (DC s.o.) : 0.10

UV/VIS (0.1 M Kaliumphosphatpuffer, pH 7.5) : $\lambda_{max}$ = 204 nm
nach Oxidation mit $H_2O_2$/Peroxidase: $\lambda_{max}$ = 574 nm

b) N-Acetyl-6-methyldihydroresorufin-4-carbonsäure-(carboxymethyl)-methylamid
Rf (DC s.o.): 0,62

UV/VIS (0.1 M Kaliumphosphatpuffer, pH 7.5) : $\lambda_{max}$ = 205 nm
nach Oxidation mit $H_2O_2$/Peroxidase : $\lambda_{max}$ = 585 nm

c) N-Acetyl-8-chlordihydroresorufin-4-carbonsäure(carboxymethyl)amid

Beispiel 3

Bestimmung von Wasserstoffperoxid

Zu einer Lösung aus

| | |
|---|---|
| Tris(hydroxymethyl)methyl-2-aminoethansulfonat | 0,1 mmol/l, pH 8 |
| Triton X 100 | 0,5 % |
| N-Acetyl-dihydroresorufin-4-carbonsäuremorpholid | 2,5 mmol/1 |
| Peroxidase | 3 U/ml |

werden unterschiedliche Mengen einer Lösung bekannten $H_2O_2$-Gehalts pipettiert, 60 min stehengelassen und danach die Extinktion bei 578 nm gemessen.

In Abb. 1 ist die Extinktion bei 578 nm gegen die $H_2O_2$-Konzentration in der Meßlösung aufgetragen. Es ergibt sich eine gute Linearität zwischen Wasserstoffperoxid-Konzentration und Extinktion.

Mit Hilfe einer so gewonnenen Eichkurve kann auch die $H_2O_2$-Konzentration in Proben mit unbekanntem $H_2O_2$-Gehalt bestimmt werden.

Beispiel 4

Bestimmung von Creatinin

Lösung 1: Tris(hydroxymethyl)methyl-2-aminoethylsulfonat
100 mmol/l, pH 8
Triton X 100 0,5 %
N-Acetyl-dihydroresorufin-4-carbonsäuremorpholid
2,5 mmol/l
Natriumchlorid 150 mmol/l
Natriumcholat 5 mmol/l
EDTA 0,5 mmol/l
Kaliumhexacyanoferrat-(II) 10 µmol/l
Natriumazid 0,2 %

Peroxidase 3 U/ml
Lipase 2 U/ml
Ascorbat-Oxidase 10 U/ml

Creatinase 6,5 U/ml
Sarcosin-Oxidase 6,5 U/ml
Creatininase 25 U/ml

Lösung 2: Creatinin-Standard-Lösung 2 mg/100 ml.

In 1 ml Lösung 1 werden 10, 20, 30, 40 und 50 µl Lösung 2 gegeben. Die Extinktion wird zeitabhängig verfolgt. Man erhält so die in Abb. 2 wiedergegebenen Kurvenverläufe, die als Eichkurven zur Bestimmung unbekannter Creatinin-Konzentrationen herangezogen werden können.

Beispiel 5

Bestimmung von Thyroxin

Lösung 1: Kaliumbarbiturat 120 mmol/l
Kaliumphosphatpuffer 18,2 mmol/l, pH 8,6
8-Anilino-1-naphthalinsulfonsäure 1,2 mmol/l
Rinderserumalbumin 0,2 %
Thyroxin-Peroxidase-Konjugat 0,5 U/l
Lösung 2: Tris/HCl 100 mmol/l, pH 8,0
Natriumperborat 3,2 mmol/l
N-Acetyl-dihydroresorufin-4-carbonsäuremorpholid
1,82 mmol/l

In ein mit Anti-Thyroxin-Antikörper beschichtetes Kunststoffröhrchen (Bindekapazität ca. 25 ng Thyroxin pro Röhrchen) gibt man 0,02 ml Thyroxin-Lösung und 1 ml Lösung 1 und laßt 30 min bei 20 - 25° C inkubieren. Der Röhrcheninhalt wird abgesaugt, einmal mit Wasser gespült und erneut abgesaugt. Danach inkubiert man 60 min mit Lösung 2, durchmischt gut und mißt die Extinktion bei 578 nm.

In Abb. 3 ist die erhaltene Eichkurve wiedergegeben.

Anhand einer solchen Eichkurve kann der unbekannte Thyroxin-Gehalt einer Probe bestimmt werden.

**Patentansprüche**

**1.** Fluorogene N-Acyl-dihydroresorufin-Derivate der allgemeinen Formel I

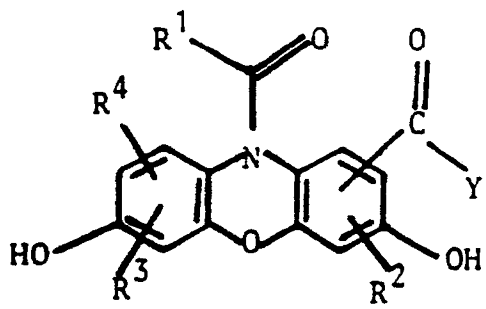

(I)

in der

| | |
|---|---|
| $R^1$ | einen Niederalkyl-, einen Phenyl- oder Naphthylrest oder Aralkylgruppe deren Arylteil eine Phenyl oder Naphthylgruppe und deren Alkylteil 1 bis 5 Kohlenstoffatome enthält, die durch Carboxyl- oder Sulfonsäurereste substituiert sein können, |
| $R^2$, $R^3$ und $R^4$, | die gleich oder verschieden sein können, Wasserstoff, Halogen, eine Niederalkyl- oder eine Niederalkoxygruppe |
| Y | die Gruppe $-NR^5R^6$ oder $-OR^7$, wobei |
| $R^5$ und $R^6$ | jeweils Wasserstoff oder eine Niederalkylgruppe, die durch Carboxyl- oder Sulfonsäurereste substituiert sein kann oder zusammen eine gegebenenfalls durch Heteroatome unterbrochene Kohlenwasserstoffbrücke und |
| $R^7$ | eine Niederalkylgruppe, die durch eine Niederalkoxy- oder eine Polyniederalkoxygruppe aus 2 bis 5 Niederalkoxyeinheiten substituiert sein kann, |

bedeuten, wobei die Niederalkyl- bzw. Niederalkoxygruppen 1 bis 7 Kohlenstoffe aufweisen.

**2.** Verfahren zur Herstellung der N-Acyl-dihydroresorufin-Derivate der allgemeinen Formel I

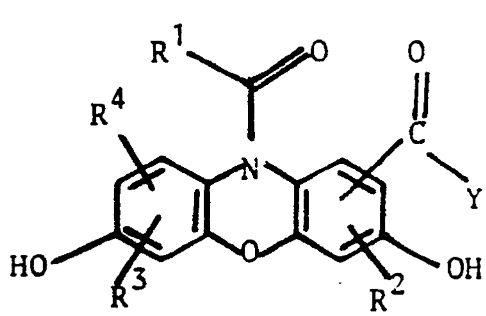

(I)

in denen $R^1$, $R^2$, $R^3$, $R^4$, Y, $R^5$, $R^6$ und $R^7$ die in Anspruch 1 angegebene Bedeutung haben, dadurch gekennzeichnet, daß man Verbindungen der allgemeinen Formel II a und II b

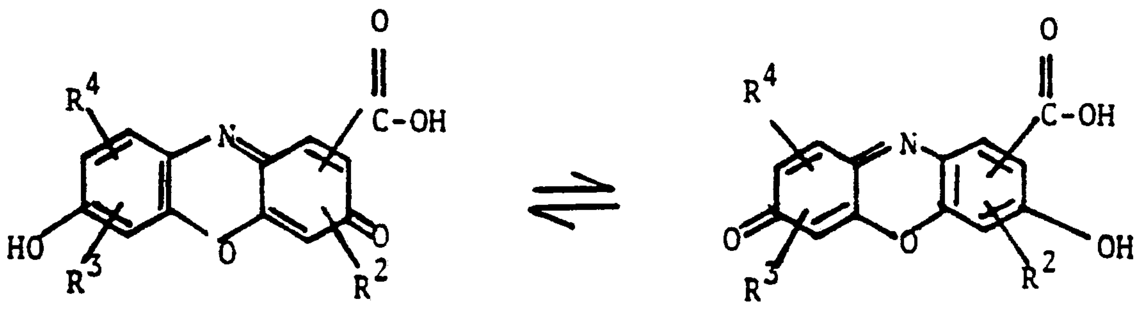

(IIa) (IIb)

in denen

$R^2$, $R^3$ und $R^4$ die in der allgemeinen Formel I angegebene Bedeutung haben, mit Zinn-II-chlorid, Chrom-II-acetat oder einem äquivalenten Reduktionsmittel oder elektrochemisch reduziert und mit einem Säureanhydrid, Säurechlorid oder äquivalenten Acylierungsmittel acyliert zu Verbindungen der allgemeinen Formel III

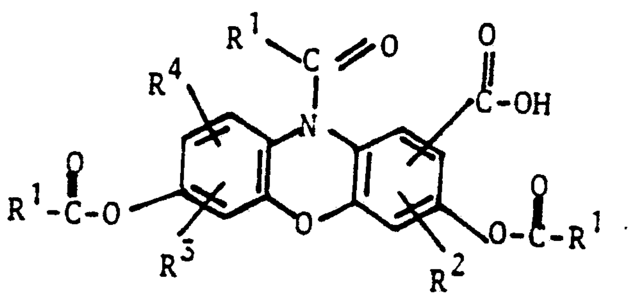

(III)

in der

$R^1$, $R^2$, $R^3$ und $R^4$ die in Formel I angegebene Bedeutung haben,

letztere mit Verbindungen der allgemeinen Formel IV

HY (IV)

in der

Y die in Formel I angegebene Bedeutung hat,

umsetzt und anschließend die den Rest $R^1$ enthaltenden O-Acylgruppen abspaltet.

3. Verfahren zur Herstellung von Dihydroresorufin-Derivaten gemäß Anspruch 2, dadurch gekennzeichnet, daß zur selektiven Hydrolyse Natriumsulfit verwendet wird.

4. Verwendung von N-Acyl-dihydroresorufin-Derivaten gemäß Anspruch 1 zum Nachweis von Wasserstoffperoxid, von peroxidatisch wirkenden Verbindungen sowie von Stoffen, die mit Sauerstoff in Gegenwart von Oxidasen unter Bildung von Wasserstoffperoxid bzw. peroxidatisch wirkenden Verbindungen reagieren, oder zum Nachweis von Peroxidasen, von Verbindungen mit Peroxidase-Aktivität sowie von Enzymaktivitäten in Wasserstoffperoxid bzw. peroxidatisch wirkende Verbindungen produzierenden Systemen.

5. Verfahren zur Bestimmung von Wasserstoffperoxid, von peroxidatisch wirkenden Verbindungen bzw. von Substanzen, die mit Sauerstoff in Gegenwart entsprechender Oxidasen Wasserstoffperoxid oder peroxidatisch wirkende Verbindungen liefern, dadurch gekennzeichnet, daß ein oder mehrere N-Acyl-dihydroresorufin-Derivate gemäß Anspruch 1, Peroxidase sowie gegebenenfalls erforderliche weitere Enzyme, ein geeignetes Puffersystem sowie gegebenenfalls weitere Reagenzien und Hilfsstoffe mit der Probe, die den zu bestimmenden Stoff enthält, vermischt und die Änderung der Absorption bzw. Fluoreszenzemission gemessen wird.

6. Verfahren zur Bestimmung von Peroxidase, von Verbindungen mit Peroxidase-Aktivität bzw. Enzymaktivitäten in Wasserstoffperoxid bzw. peroxidatisch wirkende Verbindungen produzierenden Systemen, dadurch gekennzeichnet, daß ein oder mehrere N-Acyl-dihydroresorufin-Derivate gemäß Anspruch 1, Wasserstoffperoxid bzw. eine peroxidatisch wirkende Verbindung, gegebenenfalls erforderliche Substrate und Hilfsenzyme, ein geeignetes Puffersystem sowie gegebenenfalls weitere Reagenzien und Hilfsstoffe mit der Probe, die den zu bestimmenden Stoff enthält, vermischt werden und die Änderung der Absorption bzw. Fluoreszenzemission gemessen wird.

7. Reagenz zum Nachweis von Wasserstoffperoxid, von peroxidatisch wirkenden Substanzen, bzw. von Substanzen, die mit Sauerstoff in Gegenwart entsprechender Oxidasen Wasserstoffperoxid oder peroxidatisch wirkende Verbindungen liefern, enthaltend Peroxidase, sowie gegebenenfalls erforderliche weitere Enzyme, ein oder mehrere chromogene oder/und fluorogene Substrate, ein geeignetes Puffersystem sowie gegebenenfalls weitere üblicherweise verwendete Reagenzien und Hilfsstoffe, dadurch gekennzeichnet, daß als chromogene oder/und fluorogene Substrate N-Acyl-dihydroresorufin-Derivate gemäß Anspruch 1 eingesetzt werden.

8. Reagenz zur Bestimmung von Peroxidase, von Verbindungen mit Peroxidase-Aktivität bzw. von Enzymaktivitäten in Wasserstoffperoxid bzw. peroxidatisch wirkende Verbindungen produzierenden Systemen, enthaltend Wasserstoffperoxid bzw. eine peroxidatisch wirkende Substanz, ein oder mehrere chromogene oder/und fluorogene Substrate, gegebenenfalls erforderliche Substrate und Hilfsenzyme, ein geeignetes Puffersystem sowie gegebenenfalls weitere üblicherweise verwendete Reagenzien und Hilfsstoffe, dadurch gekennzeichnet, daß als chromogene oder/und fluorogene Substrate N-Acyl-dihydroresorufin-Derivate gemäß Anspruch 1 eingesetzt werden.

9. Reagenz gemäß einem der Ansprüche 7 und 8, dadurch gekennzeichnet, daß als zusätzliche Hilfsmittel Netzmittel, Stabilisatoren, galenische Zusatzstoffe, organische Säuren oder/und Gerüstbildner verwendet werden können.

10. Reagenz gemäß einem der Ansprüche 7 bis 9, dadurch gekennzeichnet, daß es in Form einer Lösung, als Tablette, Lyophilisat, Pulvergemisch oder auf ein geeignetes Trägermaterial aufgebracht, Verwendung findet.

**Claims**

1. Fluorogenic N-acyldihydroresorufin derivatives of the general formula I

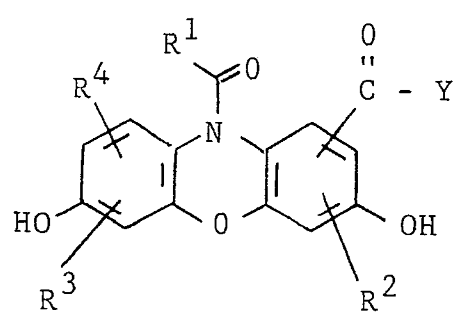

(I)

in which $R^1$ signifies a lower alkyl, a phenyl or naphthyl radical or aralkyl group, the aryl part of which contains a phenyl or naphthyl group and the alkyl part of which contains 1 to 5 carbon atoms, which can be substituted by carboxylic or sulphonic acid residues, $R^2$, $R^3$ and $R^4$, which can be the same or different, signify hydrogen, halogen, a lower alkyl or a lower alkoxy group, Y the group $-NR^5R^6$ or $-OR^7$ group, whereby $R^5$ and $R^6$ each signify hydrogen or a lower alkyl group, which can be substituted by carboxylic or sulphonic acid residues, or together a hydrocarbon bridge possibly interrupted by hetero atoms and $R^7$ a lower alkyl group which can be substituted by a lower alkoxy or poly-lower alkoxy group of 2 to 5 lower alkoxy units, whereby the lower alkyl or lower alkoxy groups have 1 to 7 carbon atoms.

2. Process for the preparation of the N-acyldihydroresorufin derivatives of the general formula I

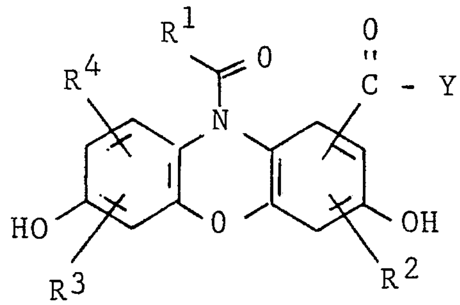

in which $R^1$, $R^2$, $R^3$, $R^4$, Y, $R^5$, $R^6$ and $R^7$ have the meaning given in claim 1, characterised in that one reduces compounds of the general formulae IIa and IIb

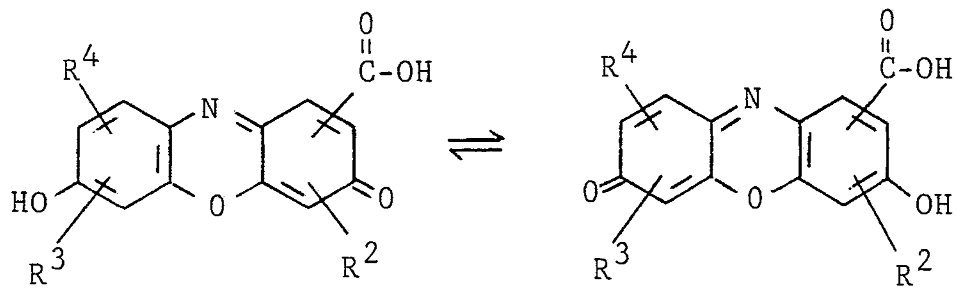

(IIa) (IIb)

in which $R^2$, $R^3$ and $R^4$ have the meaning given in the general formula I, with tin II chloride, chromium II acetate or an equivalent of reducing agent or electrochemically and acylates to compounds of the general formula III

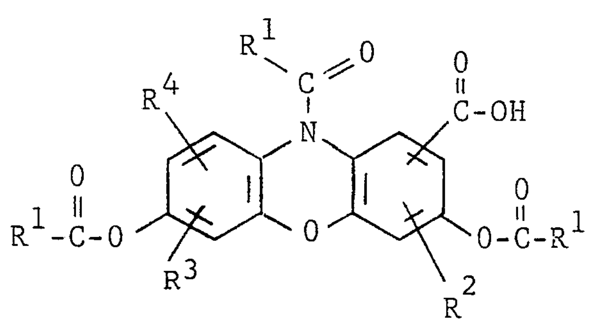

(III)

in which $R^1$, $R^2$, $R^3$ and $R^4$ have the meaning given in formula I, reacts the latter with compounds of the general formula IV

HY (IV)

in which Y has the meaning given in formula I, and subsequently splits off the O-acyl groups contained in the radical $R^1$.

3. Process for the preparation of dihydroresorufin derivatives according to claim 2, characterised in that sodium sulphite is used for the selective hydrolysis.

4. Use of N-acyldihydroresorufin derivatives according to claim 1 for the detection of hydrogen peroxide, of peroxidate-acting compounds, as well as of materials which react with oxygen in the presence of oxidases with the formation of hydrogen peroxide or of peroxidate-acting compounds or for the detection of peroxidases, of compounds with peroxidase activity, as well as of enzyme activities in systems producing hydrogen peroxide or peroxidate-acting compounds.

5. Process for the determination of hydrogen peroxide, of peroxidate-acting compounds or of substances which, with oxygen in the presence of appropriate oxidases, produce hydrogen peroxide or peroxidate-acting compounds, characterised in that one or more N-acyldihydroresorufin derivatives according to claim 1, peroxidase, as well as possibly further necessary enzymes, a suitable buffer system, as well as possibly further reagents and adjuvants, are mixed with the sample which contains the substance to be determined and the change of the absorption or fluorescence emission is measured.

6. Process for the determination of peroxidase, of compounds with peroxidase activity or of enzyme activities in systems producing hydrogen peroxide or peroxidate-acting compounds, characterised in that one or more N-acyldihydroresorufin derivatives according to claim 1, hydrogen peroxide or a peroxidate-acting compound, possibly necessary substrates and adjuvant enzymes, a suitable buffer system, as well as possibly further reagents and adjuvants are mixed with the sample which contains the substance to be determined and the change of the absorption or fluorescence emission is measured.

7. Reagent for the detection of hydrogen peroxide, of peroxidate-acting substances or of substances which provide hydrogen peroxide or peroxidate-acting compounds with oxygen in the presence of appropriate oxidases, containing peroxidase as well as further enzymes possibly necessary, one or more chromogenic and/or fluorogenic substrates, a suitable buffer system, as well as possibly further usually employed reagents and adjuvants, characterised in that N-acyldihydroresorufin derivatives according to claim 1 are used as chromogenic and/or fluorogenic substrate.

8. Reagent for the determination of peroxidase, of compounds with peroxidase activity or of enzyme activities in systems producing hydrogen peroxide or peroxidate-acting compounds, containing hydrogen peroxide or a peroxidate-acting substance, one or more chromogenic and/or fluorogenic substrates, possibly necessary substrates and adjuvant enzymes, a suitable buffer system, as well as possibly further usually employed reagents and adjuvants, characterised in that N-acyldihydroresorufin derivatives according to claim 1 are used as chromogenic and/or fluorogenic substrates.

9. Reagent according to one of claims 7 and 8, characterised in that wetting agents, stabilisers, galenical additives, organic acids and/or structure formers can be used as additional adjuvants.

10. Reagent according to one of claims 7 to 9, characterised in that it finds use in the form of a solution, as tablet, lyophilisate, powder mixture or applied to a suitable carrier material.

**Revendications**

1. Dérivés fluorogènes de N-acyl-dihydrorésorufines, de formule générale I

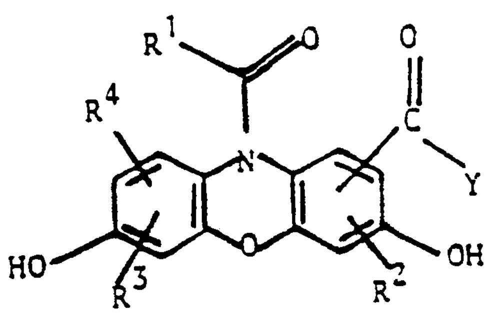

(I)

dans laquelle

| | |
|---|---|
| $R^1$ | représente un groupe alkyle inférieur, phényle ou naphtyle, ou un groupe aralkyle dont la partie aryle contient un groupe phényle ou naphtyle et dont la partie alkyle contient 1 à 5 atomes de carbone, qui peuvent être substitués par un groupe carboxyle ou acide sulfonique, |
| $R^2$, $R^3$ et $R^4$, | qui peuvent être identiques ou différents, représentent un atome d'hydrogène, d'halogène, un groupe alkyle inférieur ou alcoxy inférieur, |
| Y | représente le groupe $-NR^5R^6$ ou $-OR^7$, où |
| $R^5$ et $R^6$ | représentent chacun un atome d'hydrogène ou un groupe alkyle inférieur, qui peut être substitué par un groupe carboxyle ou acide sulfonique, ou peuvent former conjointement un pont hydrocarboné éventuellement interrompu par un hétéroatome, et |
| $R^7$ | représente un groupe alkyle inférieur, qui peut être substitué par un groupe alcoxy inférieur ou par un groupe poly(alcoxy inférieur), constitué par 2 à 5 motifs alcoxy inférieur, |

les groupes alkyle inférieur et alcoxy inférieur comportant 1 à 7 atomes de carbone.

**2.** Procédé de préparation de dérivés de N-acyl-dihydrorésorufines, de formule générale I

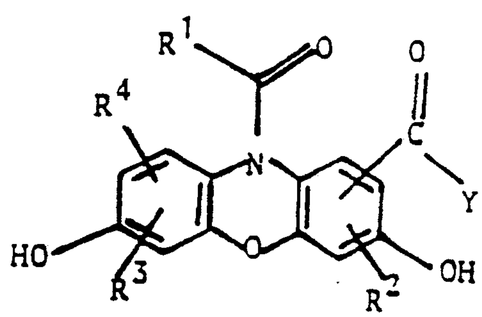

(I)

dans laquelle
$R^1$, $R^2$, $R^3$, $R^4$, Y, $R^5$, $R^6$ et $R^7$ ont les significations mentionnées dans la revendication 1, caractérisé en ce que l'on réduit des composés de formules générales IIa et IIb

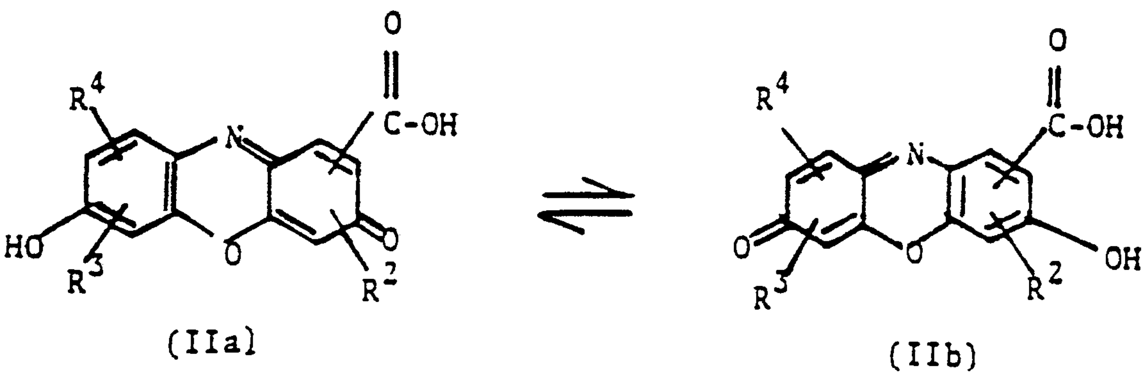

(IIa) (IIb)

dans lesquelles
$R^2$, $R^3$ et $R^4$ ont les significations mentionnées pour la formule générale I, avec du chlorure d'étain II, de l'acétate de chrome II ou un agent réducteur équivalent, ou par voie électrochimique, et à l'aide d'un anhydride d'acide, d'un chlorure d'acide ou d'un agent d'acylation équivalent, on acyle les produits en composés de formule générale III

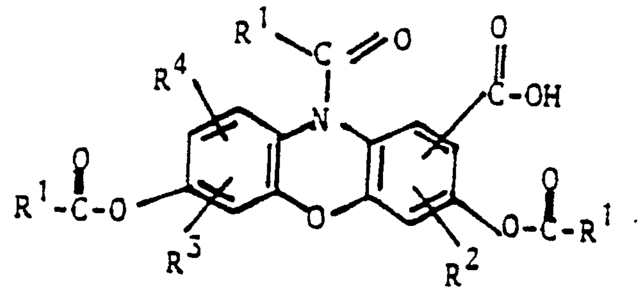

(III)

dans laquelle
$R^1$, $R^2$, $R^3$ et $R^4$ ont les significations mentionnées pour la formule I, on fait réagir ces derniers avec des composés de formule générale IV

HY (IV)

dans laquelle
Y a la signification mentionnée pour la formule I,
et ensuite, on sépare les groupes O-acyles contenant les groupes $R^1$.

3. Procédé de préparation de dérivés de dihydrorésorufines selon la revendication 2, caractérisé en ce que pour l'hydrolyse sélective, on utilise le sulfite de sodium.

4. Utilisation des dérivés de N-acyl-dihydrorésorufines selon la revendication 1, pour la détermination du peroxyde d'hydrogène, de composés ayant l'activité d'un peroxyde, ainsi que de substances réagissant avec l'oxygène, en présence d'oxydases, avec formation de peroxyde d'hydrogène ou de composés ayant l'activité d'un peroxyde, ou pour la détermination de peroxydases, de composés ayant l'activité d'une peroxydase ainsi que de l'activité enzymatique dans des systèmes produisant du peroxyde d'hydrogène ou des composés ayant l'activité d'un peroxyde.

5. Procédé pour la détermination de peroxyde d'hydrogène, de composés ayant l'activité d'un peroxyde ou de substances qui, avec de l'oxygène, en présence d'oxydases appropriées, forment du peroxyde d'hydrogène ou des composés ayant l'activité d'un peroxyde, caractérisé en ce que l'on mélange un ou plusieurs dérivés de N-acyl-dihydrorésorufines selon la revendication 1, des peroxydases ainsi que d'autres enzymes éventuellement nécessaires, un système tampon approprié ainsi qu'éventuellement d'autres réactifs et adjuvants, avec l'échantillon qui contient la substance à déterminer, et l'on mesure la modification de l'absorption ou de l'émission de fluorescence.

6. Procédé pour la détermination de peroxydases, de composés ayant l'activité d'une peroxydase ou d'une activité enzymatique dans des systèmes produisant du peroxyde d'hydrogène ou des composés ayant l'activité d'un peroxyde, caractérisé en ce que l'on mélange un ou plusieurs dérivés de N-acyl-dihydrorésorufines selon la revendication 1, du peroxyde d'hydrogène ou un composé ayant l'activité d'un peroxyde, des substrats et des enzymes auxiliaires éventuellement nécessaires, un système tampon ainsi qu'éventuellement d'autres réactifs et adjuvants nécessaires, avec l'échantillon qui contient la substance à déterminer, et l'on mesure la modification de l'absorption ou de l'émission de fluorescence.

7. Réactif pour la détermination de peroxyde d'hydrogène, de composés ayant l'activité d'un peroxyde ou de substances qui, avec de l'oxygène, en présence d'oxydases appropriées, forment du peroxyde d'hydrogène ou des composés ayant l'activité d'un peroxyde, contenant des peroxydases, ainsi que d'autres enzymes éventuellement nécessaires, un ou plusieurs substrats chromogènes et/fluorogènes, un système tampon approprié ainsi que d'autres réactifs et adjuvants usuels éventuellement nécessaires, caractérisé en ce que comme substrat chromogène et/ou fluorogène, on utilise les dérivés de N-acyl-dihydrorésorufines selon la revendication 1.

8. Réactif pour la détermination de peroxydases, de composés ayant l'activité d'une peroxydase ou

d'activités enzymatiques, dans des systèmes produisant du peroxyde d'hydrogène ou des composés ayant l'activité d'un peroxyde, contenant du peroxyde d'hydrogène ou une substance ayant l'activité d'un peroxyde, un ou plusieurs substrats chromogènes et/fluorogènes, des substrats et enzymes auxiliaires éventuellement nécessaires, un système tampon approprié ainsi qu'éventuellement d'autres réactifs et adjuvants généralement utilisés, caractérisé en ce que comme substrat chromogène et/ou fluorogène, on utilise les dérivés de N-acyl-dihydrorésorufines selon la revendication 1.

**9.** Réactif selon l'une quelconque des revendications 7 et 8, caractérisé en ce que comme adjuvants supplémentaires, on peut utiliser des agents mouillants, des stabilisants, des adjuvants galéniques, des acides organiques et/ou des agents de structure.

**10.** Réactif selon l'une quelconque des revendications 7 à 9, caractérisé en ce qu'il est utilisé sous la forme d'une solution, de pastilles, de lyophilisat, de mélange pulvérulent ou sous une forme déposée sur un support approprié.

Abbildung 1

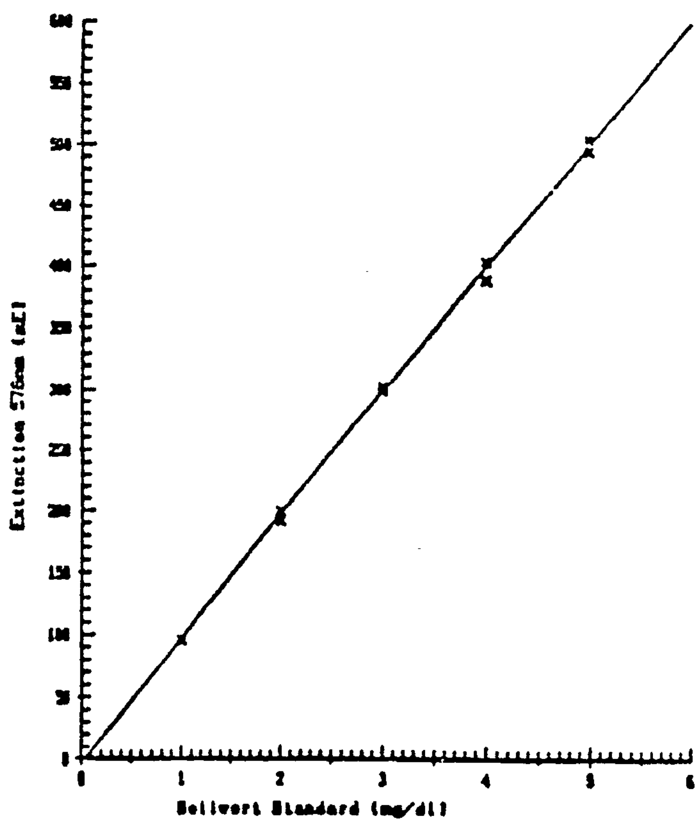

Abbildung 2

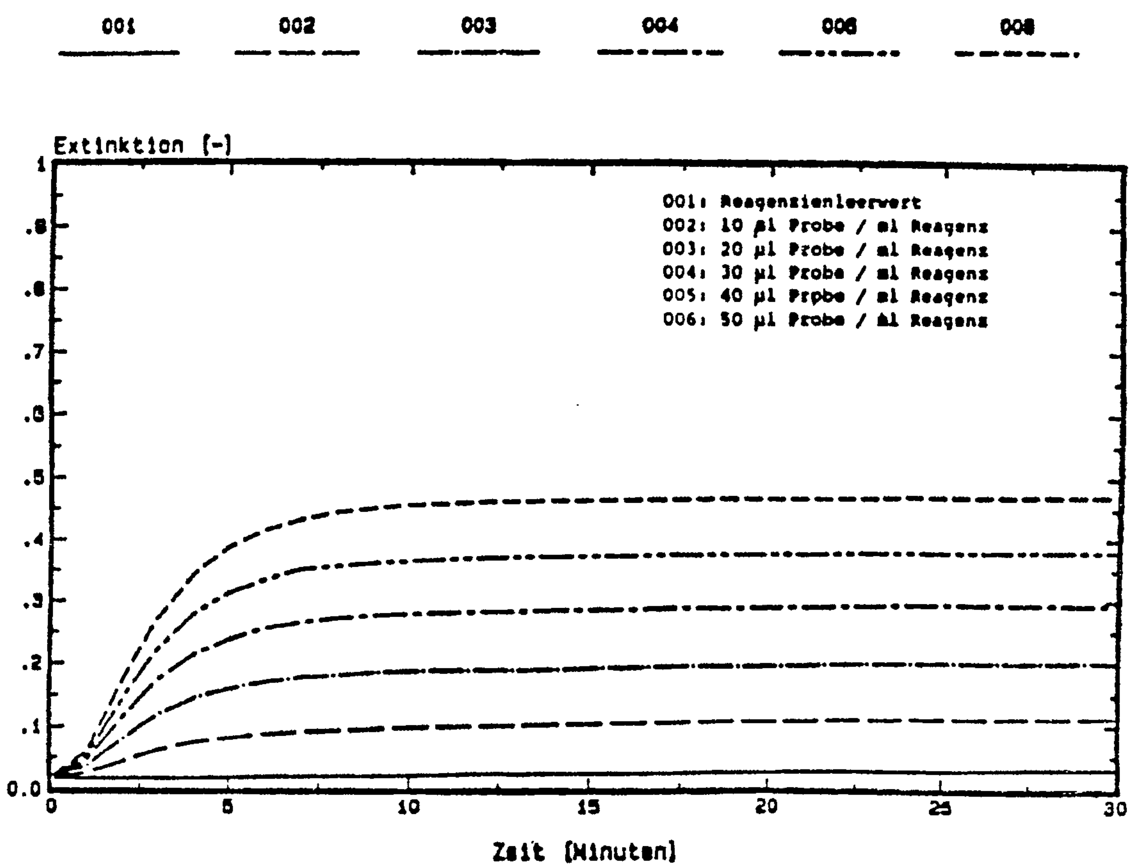
001
002
003
004
005
006
Extinktion [-]
1
.9
.8
.7
.6
.5
.4
.3
.2
.1
0.0
001: Reagenzienleerwert
002: 10 µl Probe / ml Reagenz
003: 20 µl Probe / ml Reagenz
004: 30 µl Probe / ml Reagenz
005: 40 µl Prpbe / ml Reagenz
006: 50 µl Probe / ml Reagenz
0
5
10
15
20
25
30
Zeit [Minuten]

Abbildung 3

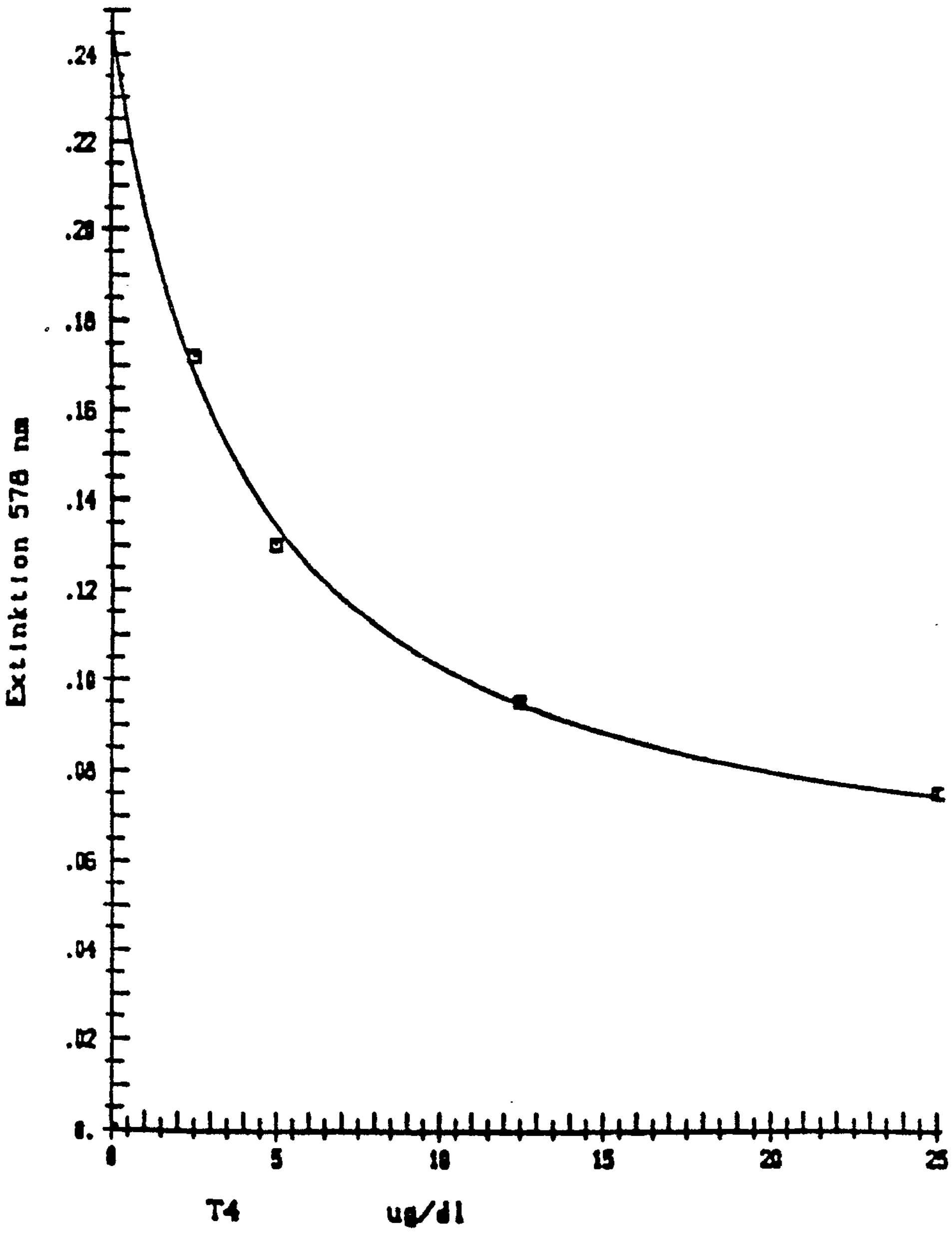
Extinktion 578 nm
.24
.22
.20
.18
.16
.14
.12
.10
.08
.06
.04
.02
0.
0
5
10
15
20
25
T4
ug/dl